# FASCICULE DE BREVET EUROPEEN

(11) **EP 4 351 475 B1**
(45) Date de publication et mention de la délivrance du brevet: **12.03.2025**
(21) Numéro de dépôt: 22732167.6
(22) Date de dépôt: 07.06.2022
(51) Int. Cl.: A61F 2/07

(54) **ENDOPROTHESE FENETREE DE L'AORTE THORACIQUE**
FENESTRIERTE ENDOPROTHESE DER AORTA THORACICA
FENESTRATED ENDOPROSTHESIS OF THE THORACIC AORTA

(30) Priorité: 10.06.2021 FR 2106128
(43) Date de publication de la demande: 17.04.2024
(73) Titulaire: UNIVERSITE CLAUDE BERNARD - LYON 1, 69100 Villeurbanne (FR); Centre National de la Recherche Scientifique, 75016 Paris (FR); Institut National de la Santé et de la Recherche Médicale, 75013 Paris (FR); Université Jean Monnet Saint-Étienne, 42100 Saint-Étienne (FR); Hospices Civils de Lyon, 69002 Lyon (FR)
(72) Inventeur: COSSET, Benoit, 69006 LYON (FR)
(74) Mandataire: IPAZ
(86) Numéro de dépôt international: PCT/EP2022/065404
(87) Numéro de publication internationale: WO 2022/258619

(56) Documents cités:
- WO-A1-2011/041773
- WO-A1-2022/053809
- US-A- 6 030 414
- US-A1- 2009 270 966
- US-A1- 2011 270 380

## Description

La présente invention se rapporte au domaine des endoprothèses, et plus particulièrement au domaine des endoprothèses aortiques thoraciques.

Un anévrisme est une dilatation localisée de la paroi d'une artère. On parle d'anévrisme lorsqu'il y a perte de parallélisme entre les bords du vaisseau et que le calibre de celui-ci est supérieur à 1,5 fois la normale.

Le risque principal est celui de la rupture de l'anévrisme, le plus souvent fatal.

Si toutes les artères peuvent être affectées, l'aorte est fréquemment touchée. Il s'agit de la plus grosse artère du corps humain.

Tous les anévrismes de l'aorte augmentent inexorablement de taille au cours du temps et le risque de rupture devient important dès que le diamètre de l'aorte dépasse 5 cm.

Toutefois, si un anévrisme est détecté à temps, il est possible de prévenir sa rupture par chirurgie ouverte, l'indication étant formelle lorsque le diamètre dépasse les 5,5 cm. Toutefois, cette intervention à l'étage thoracique est associée à une morbi-mortalité importante. Depuis le début des années 1990, un traitement moins invasif moyennant la mise en place d'une endoprothèse depuis l'artère fémorale a été proposé pour les anévrismes de l'aorte descendante : la réparation endovasculaire.

La dissection aortique correspond à une brèche dans la paroi de l'aorte, le sang s'infiltre dans la paroi et la divise en deux. Il se crée alors deux chenaux. La dissection aortique est une pathologie grave qui touche trois personnes pour 100 000 habitants. Elle peut atteindre l'aorte ascendante, l'aorte descendante ou les deux. Lorsqu'elle touche l'aorte ascendante, le traitement de référence est le remplacement de l'aorte en urgence par chirurgie ouverte. Dans l'aorte descendante, le traitement de référence est le traitement médical seul. En cas de complication est alors réalisé un traitement endovasculaire par endoprothèse.

L'endoprothèse est un stent (c'est-à dire un dispositif métallique maillé et tubulaire, communément appelé « ressort ») associé à une paroi étanche (ce que l'on appelle également « stent graft », ou «stent couvert» en français), déplié à l'intérieur de l'artère à l'emplacement de l'anévrisme. Il isole ce dernier du flux sanguin, et recrée une section cylindrique d'artère à sa place.

L'aorte se divise en deux parties, l'aorte ascendante à l'étage thoracique et l'aorte descendante à l'étage thoracique et abdominal.

Traiter les anévrismes et les dissections de l'aorte descendante par voie endovasculaire est devenu le traitement de référence.

Cependant, concernant la partie ascendante de l'aorte, le traitement de référence reste la chirurgie ouverte. Cela est principalement dû aux complexités anatomiques de la zone : l'aorte y prend la forme d'une crosse et est entourée de vaisseaux nobles ne pouvant tolérer une oblitération : ostia coronaires, troncs supra-aortiques vascularisant le cerveau.

La demande de brevet internationale W02007/028086 décrit une endoprothèse thoracique adaptée à l'arche aortique grâce à la présence de « fenêtres » dans la paroi du stent en face des trois vaisseaux, ces fenêtres permettant au sang de passer à travers la prothèse et ainsi de vasculariser les vaisseaux à destination de la tête, du cou et des bras.

Toutefois, la pose de cette endoprothèse est délicate, puisqu'il faut veiller à ce qu'elle ne tourne pas lors de son introduction, sous peine de ne pas placer les fenêtres en face des vaisseaux, de les obstruer totalement ou partiellement, ce qui serait fatal pour le patient.

De plus, le temps d'intervention lié au déploiement de l'endoprothèse reste compris entre trois à six heures, ce qui constitue une intervention lourde, sous anesthésie générale. La complexité de la procédure nécessite une implantation dans des centres experts par des opérateurs expérimentés limitant ainsi la diffusion et la démocratisation de ce traitement.

Cette intervention nécessite de placer des guides dans les vaisseaux du cou et est par conséquent associée à un risque d'AVC élevé. Demeure aussi la problématique de l'ancrage de la prothèse pour éviter son déplacement dans la direction du flux sanguin.

Enfin, ces types d'endoprothèses nécessitent d'être construites sur mesure à partir du scanner du patient et demandent ainsi un long délai de livraison (en moyenne un mois) ce qui est incompatible avec le traitement en urgence de ces pathologies.

Le document WO 2011/041773 A1 porte sur une endoprothèse dotée de deux branches latérales destinées à être insérées dans les troncs supra-aortiques. Ce type d'endoprothèse nécessite aussi d'être construit sur mesure à partir du scanner du patient et présente une durée d'intervention importante pour sa mise en place dans le corps du patient puisqu'il faut également insérer les branches dans les troncs supra-aortiques. La complexité de la procédure nécessite une implantation dans des centres experts par des opérateurs expérimentés limitant ainsi la diffusion et la démocratisation de ce traitement.

Le document US 2011/270380 A1 décrit une prothèse endovasculaire qui comprend un corps tubulaire et un raccord externe extensible depuis une configuration repliée contre le corps tubulaire à une position déployée.

Tout comme pour le document précédent, ce type d'endoprothèse nécessite une conception sur mesure et présente un temps d'intervention important du fait des ramifications qu'il présente. La complexité de la procédure nécessite une implantation dans des centres experts par des opérateurs expérimentés limitant ainsi la diffusion et la démocratisation de ce traitement.

A cet effet, la présente invention vise à permettre de traiter à l'étage thoracique les anévrismes et les dissections de l'aorte ascendante comprenant l'arche aortique, avec une sécurité maximale, tout en réduisant la complexité de l'acte chirurgical.

Plus particulièrement, l'invention a pour objet une endoprothèse fenêtrée de l'aorte thoracique comportant un premier maillage de stents, une endoprothèse tubulaire principale en tissu, ladite endoprothèse tubulaire principale étant solidaire dudit premier maillage de stents, et présentant une partie centrale arquée configurée pour être logée dans la crosse aortique d'un patient, ladite partie centrale arquée s'étendant entre une première extrémité configurée pour être logée dans une portion de l'aorte ascendante et une seconde extrémité configurée pour être logée dans une portion de l'aorte descendante, l'endoprothèse fenêtrée de l'aorte thoracique comportant en outre une collerette également en tissu et associée à un second maillage de stents, ladite collerette étant de forme sensiblement tronconiqueet dotée d'une base , ladite base de la collerette étant rapportée sur une ouverture pratiquée sur la face supérieure de la partie centrale arquée de l'endoprothèse tubulaire principale, ladite collerette étant configurée pour être logée à la base des troncs supra-aortiques du patient.

Des caractéristiques optionnelles de l'invention, complémentaires ou de substitution sont énoncées ci-après.

Selon certaines caractéristiques, le second maillage de stents peut être constitué principalement d'une maille s'étendant sur toute la circonférence de la collerette suivant des circonvolutions sensiblement sinusoïdales, la force radiale dudit maillage permettant de plaquer la collerette contre la paroi de la base des troncs supra-aortiques et ainsi de réduire le risque d'endofuites.

Selon d'autres caractéristiques, le second maillage de stents peut être constitué principalement de plusieurs mailles disjointes et radialement décalées entre elles, s'étendant sur toute la circonférence de la collerette suivant des circonvolutions sensiblement sinusoïdales, la force radiale dudit maillage permettant de plaquer la collerette contre la paroi de la base des troncs supra-aortiques et ainsi de réduire le risque d'endofuites.

Selon d'autres caractéristiques, le second maillage de stents peut comporter en outre une maille elliptique ou bien définissant le contour d'une surface paraboloïde hyperbolique, au niveau de la base de la collerette, de manière à maintenir l'ouverture pratiquée sur la face supérieure de la partie centrale arquée de l'endoprothèse tubulaire principale.

Selon d'autres caractéristiques encore, le premier maillage de stents peut être constitué principalement de plusieurs mailles disjointes, s'étendant chacune de manière circulaire sur toute la circonférence de l'endoprothèse tubulaire principale suivant des circonvolutions sensiblement sinusoïdales, la force radiale dudit maillage permettant de plaquer l'endoprothèse tubulaire principale contre la paroi aortique et ainsi de réduire le risque d'endofuites.

Selon d'autres caractéristiques encore, le premier maillage de stents peut comporter au moins une maille rattachée au second maillage de stents, de façon à empêcher les plicatures de l'endoprothèse tubulaire principale. Les plicatures correspondent à des plis formés par le corps de l'endoprothèse lors d'un mauvais déploiement de la prothèse ou lors de contraintes externes importantes, et peuvent entrainer un mauvais écoulement du flux sanguin allant de la simple sténose à l'occlusion de la prothèse.

Selon d'autres caractéristiques encore, le premier maillage de stents peut comporter au moins une maille rattachée à la maille de la base de la collerette, de façon à empêcher les plicatures de l'endoprothèse tubulaire principale.

De manière avantageuse, au moins l'une des extrémités de l'endoprothèse tubulaire principale ou bien l'extrémité libre de la collerette peut être une zone de maillage apparent dépourvue de tissu.

Le tissu recouvrant les maillages de stents peut être de préférence choisi dans la liste définie par les polyesters tressés, le Dacron tressé, le PTFE.

Les maillages de stents peuvent être de préférence en alliage à mémoire de forme, préférentiellement un alliage de Nickel-Titane.

Avantageusement, l'endoprothèse fenêtrée de l'aorte thoracique peut comprendre des marqueurs radio-opaques, préférentiellement disposés au niveau des extrémités de l'endoprothèse tubulaire principale et/ou de l'extrémité libre de la collerette.

Selon un mode de réalisation particulier, le premier maillage peut déborder sur la collerette.

Selon un autre mode de réalisation particulier, le second maillage peut déborder sur l'endoprothèse tubulaire principale.

De manière avantageuse, un renfort peut s'étendre le long de la face convexe de la partie centrale arquée de l'endoprothèse tubulaire principale, et encore plus avantageusement sur toute la longueur de ladite endoprothèse tubulaire principale.

De préférence, ce renfort est en nitinol ou bien en fil de suture.

D'autres avantages et particularités de l'invention apparaîtront à la lecture de la description détaillée de mises en œuvre et de modes de réalisation nullement limitatifs, et des dessins annexés suivants :
[Fig.1] Cette figure représente une vue schématique de profil d'une endoprothèse thoracique selon un mode de réalisation particulier de l'invention.
[Fig.2] Cette figure représente une vue schématique de profil d'une endoprothèse thoracique mis en place dans l'arche aortique d'un patient selon un mode de réalisation particulier de l'invention.

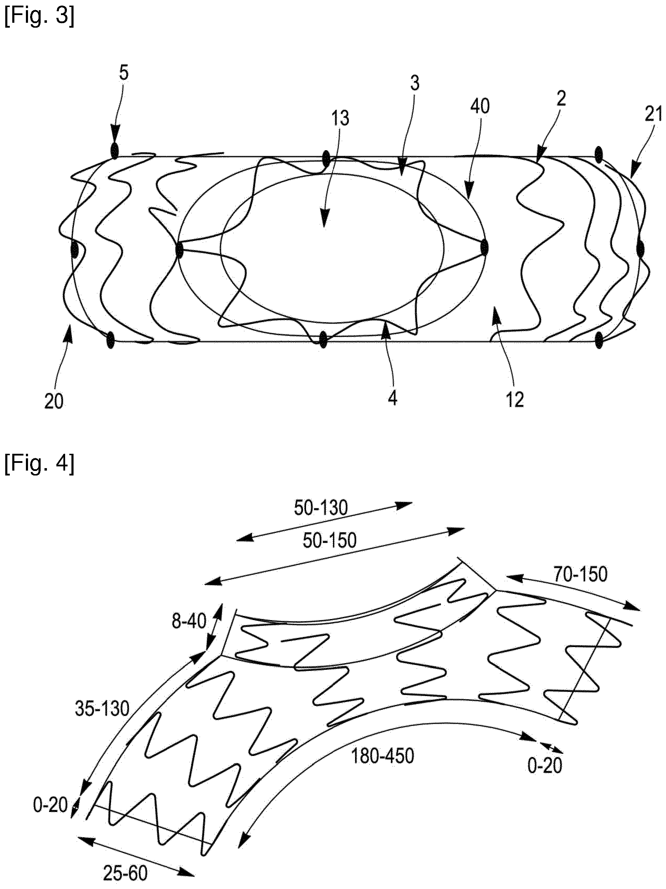

[Fig.3] Cette figure représente une vue schématique de dessus d'une endoprothèse thoracique selon un mode de réalisation particulier de l'invention.
[Fig.4] Cette figure représente une vue schématique de profil d'une endoprothèse thoracique selon l'invention accompagnée des dimensions.
[Fig.5] Cette figure représente une vue schématique de dessus d'une endoprothèse thoracique selon l'invention accompagnée des dimensions.
[Fig.6] Cette figure représente une vue schématique de profil d'une arche aortique d'un patient équipée d'une endoprothèse thoracique selon un mode de réalisation particulier de l'invention.
[Fig.7] Cette figure représente une vue schématique de profil d'une endoprothèse thoracique selon un mode de réalisation particulier de l'invention.
[Fig.8] Cette figure représente une vue schématique de dessus d'une endoprothèse thoracique selon un mode de réalisation particulier de l'invention.
[Fig.9] Cette figure représente une vue schématique de profil d'une endoprothèse thoracique mis en place dans l'arche aortique d'un patient selon un mode de réalisation particulier de l'invention.

Les modes de réalisation décrits ci-après n'étant nullement limitatifs, on pourra notamment considérer des variantes de l'invention ne comprenant qu'une sélection de caractéristiques décrites, isolées des autres caractéristiques décrites même si cette sélection est isolée au sein d'une phrase comprenant ces autres caractéristiques, si cette sélection de caractéristiques est suffisante pour conférer un avantage technique ou pour différencier l'invention par rapport à l'information de la technique antérieure.

Cette sélection comprend au moins une caractéristique, de préférence fonctionnelle sans détails structurels, ou avec seulement une partie des détails structurels si cette partie uniquement est suffisante pour conférer un avantage technique ou pour différencier l'invention par rapport à l'information de la technique antérieure.

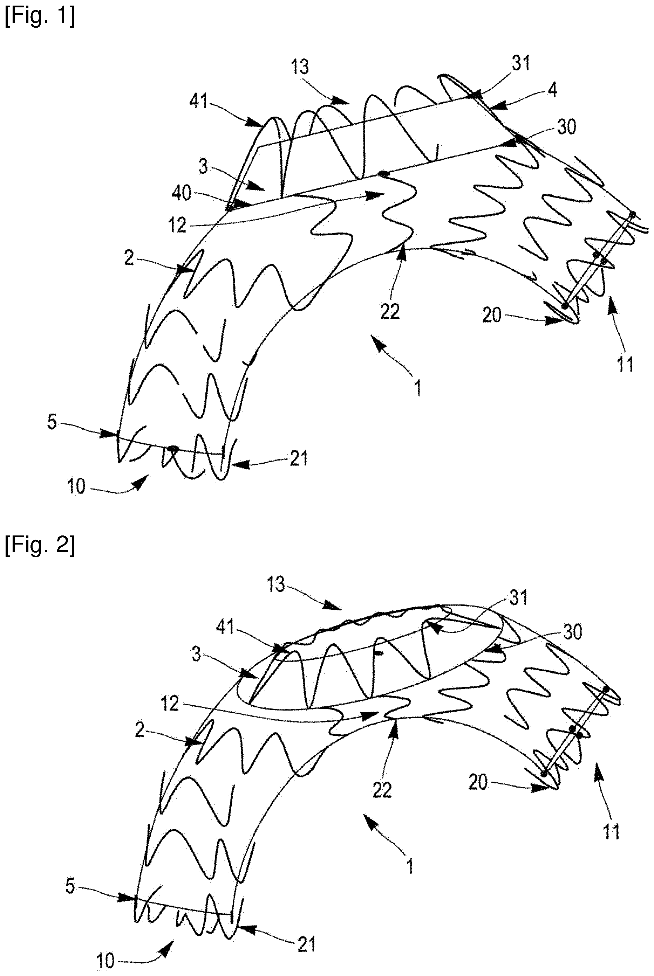

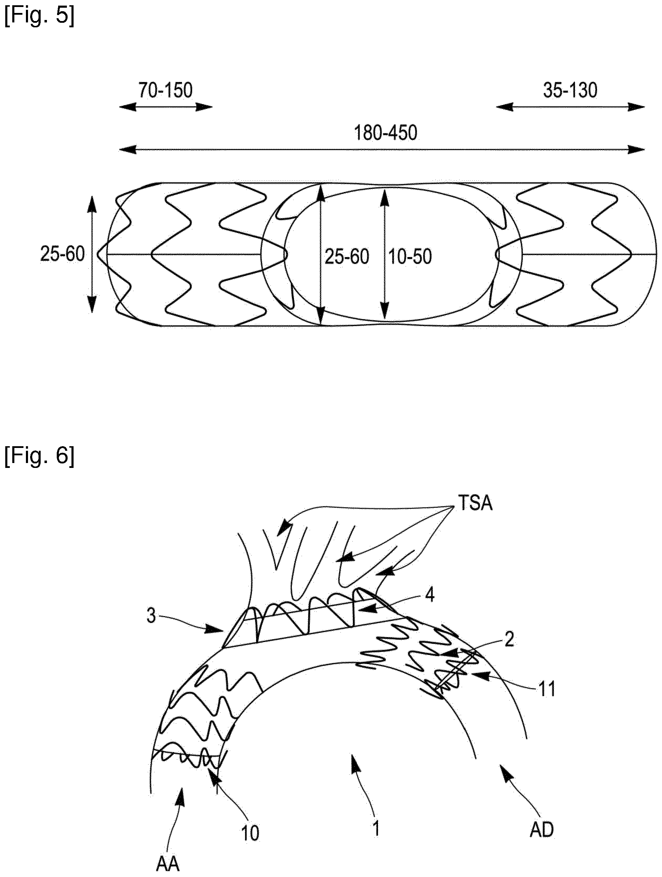

Selon l'invention et tel que représenté sur la figure 1 et sur la figure 6, l'endoprothèse fenêtrée de l'aorte thoracique comporte un premier maillage de stents 2 ainsi qu'une endoprothèse tubulaire principale 1 en tissu biocompatible formant une enveloppe étanche, l'endoprothèse tubulaire principale 1 recréant un vaisseau cylindrique et isolant le sac que forme l'anévrisme de la circulation sanguine.

L'endoprothèse tubulaire principale présente une partie centrale arquée 12 configurée pour être logée dans la crosse aortique d'un patient. Cette partie centrale arquée 12 s'étend entre une première extrémité 10 configurée pour être logée dans une portion de l'aorte ascendante AA et une seconde extrémité 11 configurée pour être logée dans une portion de l'aorte descendante AD.

Avantageusement, le tissu constituant l'endoprothèse tubulaire principale peut être choisi dans la liste définie par les polyesters tressés, le Dacron tressé, le PTFE.

Ce tissu peut par exemple être du PTFE tel que du téflon^{®} stratifié ou du dacron^{®}, ces matériaux étant communément utilisés dans les endoprothèses simples.

En outre, le diamètre extérieur et la longueur de l'endoprothèse tubulaire principale dépliée sont avantageusement choisis compris respectivement entre 25 et 60 mm et entre 180 et 450 mm. Ces dimensions permettent d'adapter parfaitement l'endoprothèse tubulaire principale 1 à la morphologie de l'arche aortique (également appelée crosse aortique) des patients.

L'invention n'est toutefois pas limitée à des dimensions ou des matériaux particuliers. L'homme de l'art saura l'adapter en fonction des cas.

Selon l'invention, l'endoprothèse tubulaire principale 1 comporte sur la face supérieure de sa partie centrale arquée 12, une ouverture 13.

On entend par « face supérieure », la portion convexe de la partie centrale arquée 12, c'est-à-dire celle qui est en regard de la base des troncs supra-aortiques.

Selon l'invention, l'endoprothèse fenêtrée de l'aorte thoracique comporte en outre une collerette 3 également en tissu et associée à un second maillage de stents 4.

Cette collerette 3 est configurée pour être logée à la base des troncs supra-aortiques TSA du patient.

On entend par « logée à la base des troncs supra-aortiques », le fait que la collerette est en regard des troncs supra-aortiques et que par conséquent, elle ne s'engage dans aucun des troncs supra-aortiques.

C'est justement cette position de la collerette au niveau de l'aorte principale qui permet "l'universalité" de l'endoprothèse ainsi que la simplicité et la rapidité d'implantation.

La collerette 3 est de forme sensiblement tronconique et sa base 30 peut être sensiblement elliptique.

La base de la collerette peut aussi définir avantageusement le contour d'une surface paraboloïde hyperbolique. Cette géométrie permet en effet une ouverture la plus large possible de la collerette, qui rend ainsi la collerette compatible avec la plupart des variations anatomiques des troncs supra-aortiques. En d'autres termes, le caractère en trois dimensions de la surface paraboloïde hyperbolique permet un élargissement maximal de l'ouverture 13 dans les trois plans de l'espace : antéro-postérieur, latéral, supéro-inférieur.Avantageusement, les dimensions des grands axes à la base et au sommet de la collerette 3 tronconique sont respectivement choisis compris entre 50 et 150 mm et entre 50 et 130 mm, afin de s'adapter parfaitement à la morphologie de l'arche aortique des patients.

De même, les dimensions des petits axes à la base et au sommet de la collerette 3 tronconique sont respectivement choisis compris entre 25 et 60 mm et entre 10 et 50 mm, afin de s'adapter parfaitement à la morphologie de l'arche aortique des patients.

La collerette 3 tronconique s'étend préférentiellement sur une longueur comprise entre 8 et 40 mm.

L'invention n'est toutefois pas limitée à des dimensions ou des matériaux particuliers. L'homme de l'art saura l'adapter en fonction des cas.

La base de la collerette 3 est rapportée sur l'ouverture 13 pratiquée sur la face supérieure de la partie centrale arquée 12 de l'endoprothèse tubulaire principale 1.

On entend par rapportée, le fait que la collerette 3 est rattachée à l'endoprothèse tubulaire principale 1 au niveau de l'ouverture 13.

L'ensemble formé par l'endoprothèse tubulaire principale et la collerette peut être réalisé en une seule pièce monobloc, par exemple par impression 3D.

Alternativement, la collerette 3 peut être suturée à l'endoprothèse tubulaire principale 1 par collage ou bien couture par exemple, l'ouverture 13 étant percée dans l'endoprothèse tubulaire principale 1 avant la suture. La suture doit garantir l'étanchéité de la jonction entre l'endoprothèse tubulaire principale 1 et la collerette 3.

La collerette 3 est de préférence constituée du même tissu que l'endoprothèse tubulaire principale 1.

L'endoprothèse tubulaire principale 1 est associée au premier maillage de stents 2, en ce sens que les stents du premier maillage sont fixés généralement sur la face externe de l'endoprothèse tubulaire principale, de sorte que les stents maintiennent l'endoprothèse tubulaire principale dans sa forme requise.

D'autres configurations non limitatives selon lesquelles, les stents sont imprégnés dans le tissu ou bien disposés contre la face interne de l'endoprothèse tubulaire principale 1, sont également envisageables.

De préférence, les stents sont en alliage à mémoire de forme, tels que par exemple les alliages de Nickel-Titane et en particulier le Nitinol.

Selon d'autres configurations non-limitatives, les stents peuvent être par exemple des Z-stents^{®} en alliage de Chrome et de Cobalt.

Avantageusement, le premier maillage de stents 2 peut être constitué principalement de plusieurs mailles disjointes, s'étendant chacune de manière circulaire sur toute la circonférence de l'endoprothèse tubulaire principale 1.

Préférentiellement, ces mailles peuvent s'étendre suivant des circonvolutions sensiblement sinusoïdales ou bien en forme de zig zag, de sorte que les mailles se déforment comme des ressorts.

Ainsi, la force radiale dudit maillage permet de plaquer l'endoprothèse tubulaire principale 1 contre la paroi aortique et permet ainsi de réduire le risque d'endofuites.

Selon d'autres configurations non-limitatives, les stents peuvent être reliés deux à deux par une pluralité de moyens de liaison. Les moyens de liaison peuvent faire l'objet de nombreux modes de réalisation, mais de façon générale peuvent consister en des barres métalliques (par exemple également en alliage de Chrome et de Cobalt, ou bien en Nitinol) fixées à leurs deux extrémités à la structure des stents, par exemple au moyen d'une boucle ou d'un crochet entourant une maille d'un stent.

Concernant la collerette 3, cette dernière est associée au second maillage de stents 4, en ce sens que les stents du second maillage sont fixés généralement sur la face externe de la collerette, de sorte que les stents maintiennent la collerette dans sa forme requise.

D'autres configurations non limitatives selon lesquelles, les stents sont imprégnés dans le tissu ou bien disposés contre la face interne de l'endoprothèse tubulaire principale 1, sont également envisageables.

De préférence, le second maillage de stents 4 est constitué principalement d'une seule maille, qui s'étend sur toute la circonférence et sur toute la longueur de la collerette 3.

Préférentiellement, cette maille peut s'étendre suivant des circonvolutions sensiblement sinusoïdales ou bien en forme de zig zag, de sorte que la maille se déforme comme un ressort.

Ainsi, la force radiale du second maillage de stents 4 permet de plaquer la collerette 3 contre la paroi de la base des troncs supra-aortiques TSA et permet ainsi de réduire le risque d'endofuites.

Tel que représenté sur la figure 2 et sur la figure 6, on voit que la paroi de la base des troncs supra-aortiques TSA tend à exercer une pression sur la collerette 3 qui à son tour exerce une contre pression contre la paroi de la base des troncs supra-aortiques, et ce grâce au second maillage de stents 4.

Selon une variante de réalisation, le second maillage de stents 4 est constitué principalement de deux mailles disjointes et radialement décalées entre elles, de manière à renforcer le maintien de la collerette produit par les deux mailles de stents.

De préférence, les stents du second maillage sont en alliage à mémoire de forme, tels que par exemple les alliages de Nickel-Titane et en particulier le Nitinol.

Selon d'autres configurations non-limitatives, les stents peuvent être par exemple des Z-stents^{®} en alliage de Chrome et de Cobalt.

Avantageusement, le second maillage de stents 4 peut comporter en outre une maille elliptique 40 au niveau de la base 30 de la collerette 3, de manière à maintenir l'ouverture 13 pratiquée sur la face supérieure de la partie centrale arquée 12 de l'endoprothèse tubulaire principale 1.

Avec cette même finalité, le second maillage de stents 4 peut comporter en outre une maille définissant le contour 40' d'une surface paraboloïde hyperbolique.

Egalement avantageusement, le premier maillage de stents 2 peut comporter au moins une maille 22 rattachée au second maillage de stents 4, de façon à empêcher les plicatures de l'endoprothèse tubulaire principale 1.

Cette maille 22 peut préférentiellement être rattachée à la maille elliptique 40 ou bien à la maille 40' définissant le contour d'une surface paraboloïde hyperbolique de la base 30 de la collerette 3, de façon à empêcher les plicatures de l'endoprothèse tubulaire principale 1.

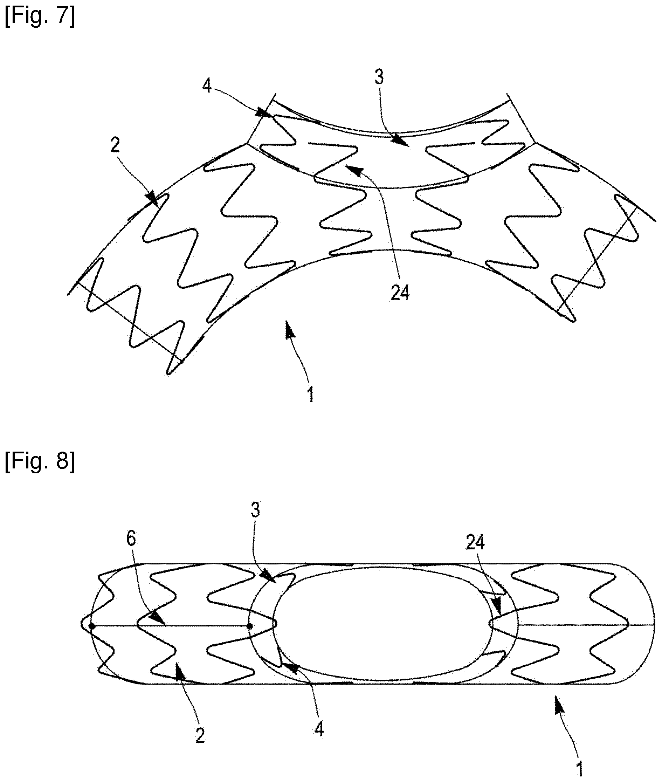

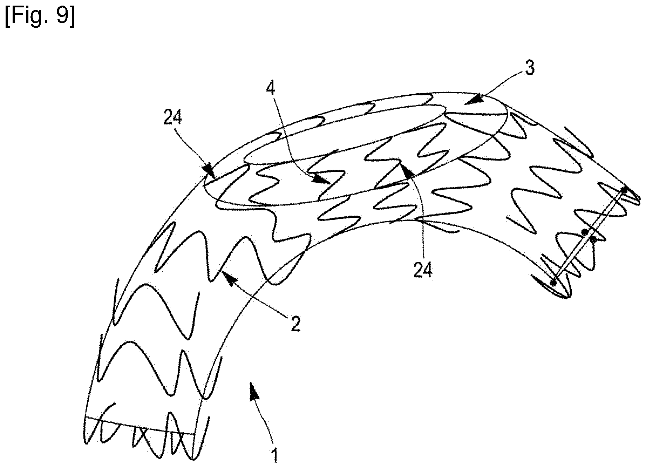

Tel que représentés sur les figures 7, 8 et 9, et de manière à maintenir la collerette 3 la plus verticale possible et ainsi empêcher son affaissement, le premier maillage 2 déborde avantageusement sur la collerette 3 sur des portions 24 de la collerette, afin de renforcer le déploiement de la collerette contre les troncs supra-aortiques.

Plus particulièrement, certaines mailles du premier maillage s'étendent jusqu'à la collerette de manière à former un cercle incomplet. Leur présence permet de limiter le maillage secondaire sur la collerette et ainsi de diminuer l'encombrement de l'endoprothèse fenêtrée. On obtient ainsi une miniaturisation de l'endoprothèse qui rend possible l'utilisation de lanceur de moindre diamètre permettant de s'introduire dans des artères fémorales de plus petit calibre.

Bien entendu, en variante et avec la même finalité, le second maillage 4 peut aussi déborder sur l'endoprothèse tubulaire principale 1 selon ce même principe.

De manière avantageuse, un renfort 6 s'étend le long de la face convexe de la partie centrale arquée 12 de l'endoprothèse tubulaire principale 1, et préférentiellement sur toute la longueur de ladite endoprothèse tubulaire principale. Ainsi, ce renfort permet d'éviter les plicatures de type « accordéon » dans le sens longitudinal, et favorise par conséquent le déploiement de l'endoprothèse sur toute sa longueur.

Ce renfort 6 peut être par exemple en nitinol ou bien constitué en fil de suture sur le tissu.

De façon à faciliter l'ancrage de l'endoprothèse tubulaire principale, l'une des extrémités de l'endoprothèse tubulaire principale peut être une zone de maillage apparent 20, 21 dépourvue de tissu appelée « free flow » en anglais.

De même, l'extrémité libre 31 de la collerette 3 peut être une zone de maillage apparent 41 dépourvue de tissu.

Ces zones de maillage apparents 20, 21 et 41 sont en fait constituées d'une maille s'étendant suivant des circonvolutions sensiblement sinusoïdales ou bien en forme de zig zag. Les sommets de la courbe sinusoïdale ou bien des zig zag peuvent alors être rapprochés ou bien écartés selon la direction radiale, c'est-à-dire la direction perpendiculaire à celle qui correspond à l'écoulement du flux sanguin.

Ces zones de maillage apparents 20, 21 et 41 s'étendent sur une distance inférieure ou égale à 20 mm.

Les sommets peuvent être rapprochés selon la direction radiale lors de la mise en place de l'endoprothèse fenêtrée dans l'aorte thoracique au moyen d'un cône et d'un guide de larguage.

Une fois le positionnement dans l'aorte arrêté, la maille tend à reprendre sa forme initiale et les sommets s'écartent selon la direction radiale pour s'ancrer dans la paroi de l'aorte ascendante AA, dans la paroi de l'aorte descendante AD et dans la paroi de la base des troncs supra-aortiques TSA.

De manière à faciliter la pose de l'endoprothèse fenêtrée de l'aorte thoracique, cette dernière peut comprendre des marqueurs radio-opaques 5, préférentiellement disposés au niveau des extrémités de l'endoprothèse tubulaire principale 1 et/ ou de l'extrémité libre 31 de la collerette 3.

Ainsi, l'invention permet d'optimiser l'ancrage de l'endoprothèse fenêtrée en évitant le déplacement de la prothèse dans la direction du flux.

Ceci est notamment renforcé par :
- Les zones de maillage apparents (appelées également free flow en anglais) au niveau des extrémités de la prothèse et au niveau de la collerette.
- Les premier et second maillages de stents orientés perpendiculairement entre eux.
- La longueur de la prothèse qui est supérieure à une endoprothèse classique (10 à 20 cm pour l'endoprothèse Gore TAG par exemple qui se trouve être la plus utilisée dans les anévrismes et les dissections de l'aorte thoracique descendante).
- La collerette 3 et le second maillage de stents 4 qui lui est associé qui tendent à se déployer contre la base de la paroi des troncs supra aortiques.

Cette endoprothèse fenêtrée permet de s'affranchir d'un grand nombre de particularités anatomiques des patients qui contre-indiquent un traitement endovasculaire avec les endoprothèses existantes (diamètre des TSA insuffisant, longueur des TSA insuffisante, localisation particulière des TSA sur l'aorte, rapports trop proches des TSA entre eux, ...).

La collerette 3 et le second maillage de stents 4 qui lui est associé permettent d'éviter la réalisation de jambages aortiques qui rendent le déploiement plus long et plus technique du fait de la cathétérisation des TSA qui consiste à placer des guides dans un vaisseau, et plus à risque de complications majeures, telles que les accidents vasculaires cérébraux.

Cette endoprothèse fenêtrée permet une rapidité d'intervention liée à la simplicité du déploiement de l'endoprothèse, soit moins d'une heure contre trois à six heures habituellement.

A noter, les différentes caractéristiques, formes, variantes et modes de réalisation de l'invention peuvent être associés les uns avec les autres, selon diverses combinaisons dans la mesure où ils ne sont pas incompatibles ou exclusifs les uns des autres.

## Revendications

1. Endoprothèse fenêtrée de l'aorte thoracique comportant un premier maillage de stents (2), une endoprothèse tubulaire principale (1) en tissu, ladite endoprothèse tubulaire principale étant solidaire dudit premier maillage de stents, et présentant une partie centrale arquée (12) configurée pour être logée dans la crosse aortique d'un patient, ladite partie centrale arquée s'étendant entre une première extrémité (10) configurée pour être logée dans une portion de l'aorte ascendante (AA) et une seconde extrémité (11) configurée pour être logée dans une portion de l'aorte descendante (AD), **caractérisé en ce que** l'endoprothèse fenêtrée de l'aorte thoracique comporte en outre une collerette (3) également en tissu et associée à un second maillage de stents (4), ladite collerette étant de forme sensiblement tronconique et dotée d'une base (30), ladite base de la collerette (3) étant rapportée sur une ouverture (13) pratiquée sur la face supérieure de la partie centrale arquée (12) de l'endoprothèse tubulaire principale (1), ladite collerette (3) étant configurée pour être logée à la base des troncs supra-aortiques (TSA) du patient.

2. Endoprothèse fenêtrée de l'aorte thoracique selon la revendication 1, **caractérisée en ce que** le second maillage de stents (4) est constitué principalement d'une maille s'étendant sur toute la circonférence de la collerette (3) suivant des circonvolutions sensiblement sinusoïdales ou en zig zag, la force radiale dudit maillage permettant de plaquer la collerette (3) contre la paroi de la base des troncs supra-aortiques (TSA) et ainsi de réduire le risque d'endofuites.

3. Endoprothèse fenêtrée de l'aorte thoracique selon la revendication 1, **caractérisée en ce que** le second maillage de stents (4) est constitué principalement de plusieurs mailles disjointes et radialement décalées entre elles, s'étendant sur toute la circonférence de la collerette (3) suivant des circonvolutions sensiblement sinusoïdales ou en zig zag, la force radiale dudit maillage permettant de plaquer la collerette (3) contre la paroi de la base des troncs supra-aortiques (TSA) et ainsi de réduire le risque d'endofuites.

4. Endoprothèse fenêtrée de l'aorte thoracique selon l'une quelconque des revendications précédentes, **caractérisée en ce que** le second maillage de stents (4) comporte en outre une maille elliptique (40) ou bien définissant le contour (40') d'une surface paraboloïde hyperbolique, au niveau de la base (30) de la collerette (3), de manière à maintenir l'ouverture (13) pratiquée sur la face supérieure de la partie centrale arquée (12) de l'endoprothèse tubulaire principale (1).

5. Endoprothèse fenêtrée de l'aorte thoracique selon l'une quelconque des revendications précédentes, **caractérisée en ce que** le premier maillage de stents (2) est constitué principalement de plusieurs mailles disjointes, s'étendant chacune sur toute la circonférence de l'endoprothèse tubulaire principale (1) suivant des circonvolutions sensiblement sinusoïdales ou en zig zag, la force radiale dudit maillage permettant de plaquer l'endoprothèse tubulaire principale contre la paroi aortique et ainsi de réduire le risque d'endofuites, et de renforcer l'ancrage de l'endoprothèse.

6. Endoprothèse fenêtrée de l'aorte thoracique selon l'une quelconque des revendications précédentes, **caractérisée en ce que** le premier maillage de stents (2) comporte au moins une maille (22) rattachée au second maillage de stents (4), de façon à empêcher les plicatures de l'endoprothèse tubulaire principale (1).

7. Endoprothèse fenêtrée de l'aorte thoracique selon la revendication 4, **caractérisée en ce que** le premier maillage de stents (2) comporte au moins une maille (22) rattachée à la maille (40, 40') de la base (30) de la collerette (3), de façon à empêcher les plicatures de l'endoprothèse tubulaire principale (1).

8. Endoprothèse fenêtrée de l'aorte thoracique selon l'une quelconque des revendications précédentes, **caractérisée en ce qu'**au moins l'une des extrémités de l'endoprothèse tubulaire principale (1) ou bien l'extrémité libre de la collerette (3) est une zone de maillage apparent (20, 21, 41) dépourvue de tissu.

9. Endoprothèse fenêtrée de l'aorte thoracique selon l'une quelconque des revendications précédentes, **caractérisée en ce que** les maillages de stents sont en alliage à mémoire de forme, préférentiellement un alliage de Nickel-Titane, et le tissu recouvrant les maillages de stents est choisi dans la liste définie par les polyesters tressés, le Dacron tressé, le PTFE.

10. Endoprothèse fenêtrée de l'aorte thoracique selon l'une quelconque des revendications précédentes, **caractérisée en ce qu'**elle comprend des marqueurs radio-opaques (5), préférentiellement disposés au niveau des extrémités de l'endoprothèse tubulaire principale (1) et de l'extrémité libre (31) de la collerette (3).

11. Endoprothèse fenêtrée de l'aorte thoracique selon l'une quelconque des revendications précédentes, **caractérisée en ce que** le premier maillage (2) déborde sur la collerette (3).

12. Endoprothèse fenêtrée de l'aorte thoracique selon l'une quelconque des revendications précédentes, **caractérisée en ce que** le second maillage (4) déborde sur l'endoprothèse tubulaire principale (1).

13. Endoprothèse fenêtrée de l'aorte thoracique selon l'une quelconque des revendications précédentes, **caractérisée en ce qu'**un renfort (6) s'étend le long de la face convexe de la partie centrale arquée (12) de l'endoprothèse tubulaire principale (1), et préférentiellement sur toute la longueur de ladite endoprothèse tubulaire principale.

14. Endoprothèse fenêtrée de l'aorte thoracique selon la revendication précédente, **caractérisée en ce que** le renfort est en nitinol ou constitué en fil de suture sur le tissu.

## Patentansprüche

1. Fenestrierte Endoprothese der Aorta Thoracica, die aufweist ein erstes Stent-Netz (2), eine rohrförmige Hauptendoprothese (1) aus Gewebe, wobei die rohrförmige Hauptendoprothese mit dem ersten Stent-Netz fest verbunden ist und einen bogenförmigen Mittelteil (12) aufweist, der so ausgebildet ist, dass er sich im Aortenbogen eines Patienten befindet, wobei sich der bogenförmige Mittelteil zwischen einem ersten Ende (10), das so ausgebildet ist, dass es sich in einem Abschnitt der aufsteigenden Aorta (AA) befindet, und einem zweiten Ende (11) erstreckt, das so ausgebildet ist, dass es sich in einem Abschnitt der absteigenden Aorta (AD) befindet, **dadurch gekennzeichnet, dass** die fenestrierte Endoprothese der Aorta Thoracica ferner einen Kragen (3) ebenfalls aus Gewebe aufweist, der mit einem zweiten Stent-Netz (4) verbunden ist, wobei der Kragen im Wesentlichen kegelstumpfförmig und mit einer Basis (30) versehen ist, wobei die Basis des Kragens (3) auf eine Öffnung (13) aufgesetzt ist, die auf der Oberseite des bogenförmige Mittelteils (12) der röhrenförmigen Hauptendoprothese (1) vorgesehen ist, wobei der Kragen (3) so ausgebildet ist, dass er sich an der Basis der supraaortalen Äste (TSA) des Patienten befindet.

2. Fenestrierte Endoprothese der Aorta Thoracica nach Anspruch 1, **dadurch gekennzeichnet, dass** das zweite Stent-Netz (4) hauptsächlich aus einer Masche besteht, die sich über den gesamten Umfang des Kragens (3) in im Wesentlichen sinusförmigen oder zickzackförmigen Windungen erstreckt, wobei durch die radiale Kraft des Netzes der Kragen (3) gegen die Wand der Basis der supraaortalen Äste (TSA) gedrückt werden kann und so das Risiko von Endoleckagen verringert werden kann.

3. Fenestrierte Endoprothese der Aorta Thoracica nach Anspruch 1, **dadurch gekennzeichnet, dass** das zweite Stent-Netz (4) hauptsächlich aus einer Vielzahl von getrennten und radial zueinander versetzten Maschen besteht, die sich über den gesamten Umfang des Kragens (3) in im Wesentlichen sinusförmigen oder zickzackförmigen Windungen erstrecken, wobei durch die radiale Kraft des Netzes der Kragen (3) gegen die Wand der Basis der supraaortalen Äste (TSA) gedrückt werden kann und so das Risiko von Endoleckagen verringert werden kann.

4. Fenestrierte Endoprothese der Aorta Thoracica nach einem der vorstehenden Ansprüche, **dadurch gekennzeichnet, dass** das zweite Stent-Netz (4) ferner eine elliptische Masche (40) oder eine Masche (40'), welche die Kontur (40') einer hyperbolischen Paraboloiden klar definiert, an der Basis (30) des Kragens (3) aufweist, um die Öffnung (13) beizubehalten, die auf der Oberseite des bogenförmigen Mittelteils (12) der rohrförmigen Hauptendoprothese (1) vorgesehen ist.

5. Fenestrierte Endoprothese der Aorta Thoracica nach einem der vorstehenden Ansprüche, **dadurch gekennzeichnet, dass** das erste Stent-Netz (2) hauptsächlich aus einer Vielzahl von getrennten Maschen besteht, die sich jeweils um den gesamten Umfang der rohrförmigen Hauptendoprothese (1) in im Wesentlichen sinusförmigen oder zickzackförmigen Windungen erstrecken, wobei durch die radiale Kraft des Netzes die rohrförmige Hauptendoprothese gegen die Aortenwand gedrückt werden kann und so das Risiko von Endoleckagen verringert werden kann, und die Verankerung der Endoprothese verstärkt werden kann.

6. Fenestrierte Endoprothese der Aorta Thoracica nach einem der vorstehenden Ansprüche, **dadurch gekennzeichnet, dass** das erste Stent-Netz (2) mindestens eine Masche (22) aufweist, die mit dem zweiten Stent-Netz (4) so in Verbindung gebracht ist, dass das Abknicken der rohrförmigen Hauptendoprothese (1) verhindert wird.

7. Fenestrierte Endoprothese der Aorta Thoracica nach Anspruch 4, **dadurch gekennzeichnet, dass** das erste Stent-Netz (2) mindestens eine Masche (22) aufweist, die so mit der Masche (40, 40') der Basis (30) des Kragens (3) in Verbindung gebracht ist, dass das Abknicken der rohrförmigen Hauptendoprothese (1) verhindert wird.

8. Fenestrierte Endoprothese der Aorta Thoracica nach einem der vorstehenden Ansprüche, **dadurch gekennzeichnet, dass** mindestens eines der Enden der rohrförmigen Hauptendoprothese (1) bzw. das freie Ende des Kragens (3) ein Bereich mit sichtbarem Netz (20, 21, 41) ist, das frei von Gewebe ist.

9. Fenestrierte Endoprothese der Aorta Thoracica nach einem der vorstehenden Ansprüche, **dadurch gekennzeichnet, dass** die Stent-Netze aus einer Formgedächtnislegierung, vorzugsweise einer Nickel-Titan-Legierung, bestehen und das Gewebe, mit dem die Stent-Netze bedeckt sind, aus der Liste ausgewählt wird bestehend aus geflochtenem Polyester, geflochtenem Dacron, PTFE.

10. Fenestrierte Endoprothese der Aorta Thoracica nach einem der vorstehenden Ansprüche, **dadurch gekennzeichnet, dass** sie radio-opake Marker (5) aufweist, die vorzugsweise an den Enden der rohrförmigen Hauptendoprothese (1) und dem freien Ende (31) des Kragens (3) angeordnet sind.

11. Fenestrierte Endoprothese der Aorta Thoracica nach einem der vorstehenden Ansprüche, **dadurch gekennzeichnet, dass** das erste Netz (2) über den Kragen (3) hinausgeht.

12. Fenestrierte Endoprothese der Aorta Thoracica nach einem der vorstehenden Ansprüche, **dadurch gekennzeichnet, dass** das zweite Netz (4) über die rohrförmige Hauptendoprothese (1) hinausgeht.

13. Fenestrierte Endoprothese der Aorta Thoracica nach einem der vorstehenden Ansprüche, **dadurch gekennzeichnet, dass** sich eine Verstärkung (6) entlang der konvexen Fläche des bogenförmigen Mittelteils (12) der rohrförmige Hauptendoprothese (1) erstreckt, und vorzugsweise über die gesamte Länge der rohrförmigen Hauptendoprothese.

14. Fenestrierte Endoprothese der Aorta Thoracica nach einem der vorstehenden Ansprüche, **dadurch gekennzeichnet, dass** die Verstärkung aus Nitinol oder aus Nähfäden aus dem Gewebe besteht.

## Claims

1. A fenestrated endoprosthesis of the thoracic aorta comprising a first stent meshing (2), a main tubular endoprosthesis (1) made of fabric, said main tubular endoprosthesis being integral with said first stent meshing, and having an arcuate central part (12) configured to be housed in the aortic arch of a patient, said arcuate central part extending between a first end (10) configured to be housed in a portion of the ascending aorta (AA) and a second end (11) configured to be housed in a portion of the descending aorta (AD), **characterised in that** the fenestrated endoprosthesis of the thoracic aorta further includes a flange (3) also made of fabric and associated with a second stent meshing (4), said flange being substantially frustoconical in shape and provided with a base (30), said base of the flange (3) being fitted over an aperture (13) made on the upper face of the arcuate central part (12) of the main tubular endoprosthesis (1), said flange (3) being configured to be housed at the base of the patient's supra-aortic trunks (TSA).

2. The fenestrated endoprosthesis of the thoracic aorta according to claim 1, **characterised in that** the second stent meshing (4) mainly consists of a mesh extending over the entire circumference of the flange (3) in substantially sinusoidal or zigzag circumvolutions, the radial force of said meshing making it possible to press the flange (3) against the wall of the base of the supra-aortic trunks (TSA) and thus reduce the risk of endoleaks.

3. The fenestrated endoprosthesis of the thoracic aorta according to claim 1, **characterised in that** the second stent meshing (4) mainly consists of a plurality of disjointed meshes radially offset from one another, extending over the entire circumference of the flange (3) in substantially sinusoidal or zigzag circumvolutions, the radial force of said meshing making it possible to press the flange (3) against the wall of the base of the supra-aortic trunks (TSA) and thus reduce the risk of endoleaks.

4. The fenestrated endoprosthesis of the thoracic aorta according to any of the preceding claims, **characterised in that** the second stent meshing (4) further includes an elliptical mesh (40) or a mesh defining the contour (40') of a hyperbolic paraboloid surface, at the base (30) of the flange (3), so as to maintain the aperture (13) made on the upper face of the arcuate central part (12) of the main tubular endoprosthesis (1).

5. The fenestrated endoprosthesis of the thoracic aorta according to any of the preceding claims, **characterised in that** the first stent meshing (2) mainly consists of a plurality of disjointed meshes, each extending over the entire circumference of the main tubular endoprosthesis (1) in substantially sinusoidal or zigzag circumvolutions, the radial force of said meshing making it possible to press the main tubular endoprosthesis against the aortic wall and thus reduce the risk of endoleaks, and reinforce the anchoring of the endoprosthesis.

6. The fenestrated endoprosthesis of the thoracic aorta according to any of the preceding claims, **characterised in that** the first stent meshing (2) includes at least one mesh (22) attached to the second stent meshing (4), so as to prevent plications of the main tubular endoprosthesis (1).

7. The fenestrated endoprosthesis of the thoracic aorta according to claim 4, **characterised in that** the first stent meshing (2) includes at least one mesh (22) attached to the mesh (40, 40') of the base (30) of the flange (3), so as to prevent plications of the main tubular endoprosthesis (1).

8. The fenestrated endoprosthesis of the thoracic aorta according to any of the preceding claims, **characterised in that** at least one of the ends of the main tubular endoprosthesis (1) or the free end of the flange (3) is an apparent meshing zone (20, 21, 41) devoid of fabric.

9. The fenestrated endoprosthesis of the thoracic aorta according to any of the preceding claims, **characterised in that** the stent meshings are made of a shape memory alloy, preferably a Nickel-Titanium alloy, and the fabric covering the stent meshings is selected from the list defined by braided polyesters, braided Dacron, PTFE.

10. The fenestrated endoprosthesis of the thoracic aorta according to any of the preceding claims, **characterised in that** it comprises radiopaque markers (5), preferably disposed at the ends of the main tubular endoprosthesis (1) and at the free end (31) of the flange (3).

11. The fenestrated endoprosthesis of the thoracic aorta according to any of the preceding claims, **characterised in that** the first meshing (2) projects onto the flange (3).

12. The fenestrated endoprosthesis of the thoracic aorta according to any of the preceding claims, **characterised in that** the second meshing (4) projects onto the main tubular endoprosthesis (1).

13. The fenestrated endoprosthesis of the thoracic aorta according to any of the preceding claims, **characterised in that** a reinforcement (6) extends along the convex face of the arcuate central part (12) of the main tubular endoprosthesis (1), and preferably over the entire length of said main tubular endoprosthesis.

14. The fenestrated endoprosthesis of the thoracic aorta according to the preceding claim, **characterised in that** the reinforcement is made of nitinol or consists of suture wire on the fabric.
